# EUROPEAN PATENT SPECIFICATION

(11) **EP 1 348 439 B1**
(45) Date of publication and mention of the grant of the patent: **10.08.2005**
(21) Application number: 03006637.7
(22) Date of filing: 25.03.2003
(51) Int. Cl.: A61K 35/78, A61P 15/02, A61P 15/12

(54) **Compositions for vaginal use**
Zusammensetzungen für vaginale Verwendung
Compositions pour utilisation vaginale

(30) Priority: 29.03.2002 IT MI20020665
(43) Date of publication of application: 01.10.2003
(73) Proprietor: MARFARMA HOLDING S.p.A., 20154 Milano (IT)
(72) Inventor: Heyda, Alessandro, 20145 Milano (IT)
(74) Representative: Minoja, Fabrizio, Dr.

(56) References cited:
- WO-A-00/03684
- US-A- 5 122 374
- "TEPESCOHUITE EXTRACTS" SOAP COSMETICS CHEMICAL SPECIALTIES, MAC.NAIR-DORLAND CO. NEW YORK, US, vol. 69, no. 10, 1 October 1993 (1993-10-01), page 109 XP000431371 ISSN: 0091-1372
- "LE TEPEZCOHUITE LE CONQUISTADOR DE LA BEAUTE" PARFUMS, COSMETIQUES, AROMES, SOCIETE D'EXPANSION TECHNIQUE ET ECONOMIQUE S.A. PARIS, FR, no. 90, 1 December 1989 (1989-12-01), pages 93-94, XP000113283 ISSN: 0337-3029

## Description

The present invention relates to vaginal or feminine intimate hygiene compositions containing active ingredients of vegetable origin.

More particularly, the present invention relates to fluid compositions in the form of gel, creams or vaginal irrigations containing isoflavones, in particular soy isoflavones, and a *Mimosa tenuiflora* extract.

The present invention also relates to the use of combinations of isoflavones, in particular soy isoflavones, and a *Mimosa tenuiflora* extract, for the preparation of cosmetics or medicaments for the topical treatment of postmenopausal symptoms, in particular for the treatment of vaginal dryness and atrophic vaginitis.

*Mimosa tenuiflora* (Willd.) Poiret is a shrub belonging to the class of *Mimosaceae-Fabaceae.* It grows spontaneously in Central America, where it is called Tepezcohuite (skin tree). The drug, obtained from the bark, has been used for centuries in traditional medicine for the treatment of burns and of skin inflammatory and phlogistic conditions. The bark of *Mimosa tenuiflora* contains, in fact, triterpene saponins having cytotrophic activity, as well as remarkable amounts of polyphenols, bioflavonoids, tannins and starch, which are known to favorably act in the treatment of burns and in the regeneration of the skin (Anton R. et al, Journal of Ethnopharmacology, 38 (1993), 153-157).

*Mimosa tenuiflora* extract (aqueous, alcoholic or glycolic) is therefore useful in the treatment of burns and irritative and inflammatory conditions of the skin, as it exerts trophic, cicatrizing, protective, antiradicalic, antihyaluronidase and antibacterial actions against microorganisms present on the skin, in particular against yeasts and dermatophites (Lozoya X. Et al, Archiv. Invest. Med. (Méx), Vol. 20, N. 1, 1989, 87-93).

Hormone replacement therapy (with estrogens and optionally progestogens) used in the treatment of post-menopausal disorders remains controversial. It is in fact known that, while on the one hand it involves a series of benefits (such as reduction of the risks of cardiovascular diseases, osteoporosis, colon cancer, reduction of hot flashes, anxiety, depression, vaginal atrophy and dryness, improvement in the general conditions of the skin and of the muscle tone), on the other hand it induces an increase by 32 to 46% of the risk of breast cancer (New England Journal of Medicine, Vol. 332:1589-1593, June 15, 1994, N. 24) and a moderate increase in the risks of venous thromboembolia.

Soy isoflavones are phytoestrogens studied for some time due to their estrogenic and antioxidant actions. Epidemiological comparison studies between western and eastern female population have shown in fact that a diet rich in phytoestrogens improves symptoms due to lack of estrogens of the women in menopause (Lock. M., Cult. Med. Psychiatry, 1986; 10; 23-46) and can protect against breast cancer, osteoporosis and cardiovascular diseases (Adlercreutz H., Scand. J. Clin. Lab. Invest., 1990: 50 (Suppl. 210): 3-23; and Rose D.P., et al, Cancer 1986; 58: 2363-2371).

The compositions of the present invention, containing isoflavones preferably soy isoflavones and a *Mimosa tenuiflora* extract, exert favorable activities on the vaginal epithelium, in particular antiinflammatory, antibacterial, riepithelizing, protective and antioxidant actions, and are therefore useful for the treatment of vaginal dryness and atrophic vaginitis conditions during menopause.

In particular, it has been found that the compositions of the present invention have surprisingly higher activity than that predictable considering the activity of the single active substances, thus proving the synergistic effect of the components.

According to the present invention, the *Mimosa tenuiflora* extract is preferably a glycolic extract of the bark, having a content in polyphenols higher than 5% by weight and a content in bioflavonoids higher than 1%. Said extract will preferably be present in the compositions of the invention in amounts ranging from 0.1 to 20% by weight.

According to the present invention, soy isoflavones are preferably selected from genistein, daidzein, glycitein and their glycosides genistin, daidzin, and glycitin, and they will be present in amounts preferably ranging from 0.01 to 3% by weight.

The compositions used in the present invention will be formulated in the form of gel, creams, solutions, vaginal tablets or capsules, vaginal irrigations, using conventional techniques and excipients, such as diluents, disintegrants, moisturizers and lubricants. According to the present invention, a preferred formulation is that in gel.

An example of formulation according to the invention is reported in the following.

### EXAMPLE

| Gel formulation. | |
|---|---|
| Ingredient | Amount % by weight |
| *Mimosa tenuiflora* glycolic extract | 1 |
| Soy isoflavonoids | 0.10 |
| Peg-7 glyceryl cocoate | 47 |
| Acrylate/stearate-20 | 6.50 |
| methacrylate copolymer | |
| Propylene glycol | 3 |
| Peg-40 hydrogenated castor oil | 3 |
| Imidazolidinylurea | 0.30 |
| Sodium hydroxide | 0.13 |
| Methylchloroisothiazolinone- | 0.05 |
| methylisothiazolinone | |
| Water | q.s. to 100 |

## Claims

1. Fluid compositions in the form of gel, creams or vaginal irrigations, containing isoflavones and a *Mimosa tenuiflora* extract.

2. Compositions as claimed in claim 1, wherein isoflavones are soy isoflavones.

3. Compositions as claimed in claim 1 wherein the *Mimosa tenuiflora* extract is a glycolic extract of the bark.

4. Compositions as claimed in claim 3 wherein the extract has a content in polyphenols higher than 5% by weight and a content in bioflavonoids higher than 1% by weight.

5. Compositions as claimed in claim 1 and 2, wherein soy isoflavones are selected from genistein, daidzein, glycitein, genistin, daidzin and glycitin.

6. Compositions as claimed in any one of claims 1 to 5, in the form of gel.

7. Compositions as claimed in any one of claims 1 to 6, containing 0.01 to 3% by weight of isoflavones and 0.1 to 20% by weight of *Mimosa tenuiflora* extract.

8. The use of soy isoflavones and a *Mimosa tenuiflora* extract in combination for the preparation of cosmetics or medicaments for the intimate feminine hygiene and for the topical treatment of vaginal climacteric symptoms.

## Patentansprüche

1. Fluidzusammensetzungen in Gelform, in Form von Cremes oder Vaginalspülungen, die Isoflavone und einen Mimosa tenuiflora-Extrakt enthalten.

2. Zusammensetzungen nach Anspruch 1, wobei Isoflavone Sojaisoflavone sind.

3. Zusammensetzungen nach Anspruch 1, wobei der Mimosa tenuiflora-Extrakt ein glykolischer Extrakt der Rinde ist.

4. Zusammensetzungen nach Anspruch 3, wobei der Extrakt einen Gehalt an Polyphenolen von mehr als 5 Gew.-% und einen Gehalt an Bioflavonoiden von mehr als 1 Gew.-% aufweist.

5. Zusammensetzungen nach Anspruch 1 und Anspruch 2, wobei die Sojaisoflavone aus Genistein, Daidzein, Glycitein, Genistin, Daidzin und Glycitin ausgewählt sind.

6. Zusammensetzungen nach einem der Ansprüche 1 bis 5 in Gelform.

7. Zusammensetzungen nach einem der Ansprüche 1 bis 6, die 0,01 bis 3 Gew.-% Isoflavone und 0,1 bis 20 Gew.-% Mimosa tenuiflora-Extrakt enthalten.

8. Verwendung von Sojaisoflavonen und eines Mimosa tenuiflora-Extrakts in Kombination für die Herstellung von Kosmetika oder Arzneimitteln für die weibliche Intimhygiene und für die topische Behandlung von vaginalen klimakterischen Symptomen.

## Revendications

1. Compositions fluides sous la forme de gel, de crèmes ou d'agents d'irrigation vaginale, contenant des isoflavones et un extrait de *Mimosa tenuiflora*.

2. Compositions telles que revendiquées dans la revendication 1, dans lesquelles les isoflavones sont des isoflavones de soja.

3. Compositions telles que revendiquées dans la revendication 1, dans lesquelles l'extrait de *Mimosa tenuiflora* est un extrait glycolique de l'écorce.

4. Compositions telles que revendiquées dans la revendication 3, dans lesquelles l'extrait a une teneur en polyphénols supérieure à 5% en poids et une teneur en bioflavonoïdes supérieure à 1% en poids.

5. Compositions telles que revendiquées dans la revendication 1 et 2, dans lesquelles les isoflavones de soja sont choisies parmi la génistéine, la daidzéine, la glycitéine, la génistine, la daidzine et la glycitine.

6. Compositions telles que revendiquées dans l'une quelconque des revendications 1 à 5, sous la forme de gel.

7. Compositions telles que revendiquées dans l'une quelconque des revendications 1 à 6, contenant 0,01 à 3% en poids d'isoflavones et 0,1 à 20% en poids d'extrait de *Mimosa tenuiflora*.

8. Utilisation d'isoflavones de soja et d'un extrait de *Mimosa tenuiflora* en combinaison pour la préparation de cosmétiques ou de médicaments pour l'hygiène intime féminine et pour le traitement topique de symptômes climatères vaginaux.
